(19) Europäisches Patentamt
European Patent Office
Office européen des brevets

(11) Veröffentlichungsnummer: **0 244 360 B1**

(12) **EUROPÄISCHE PATENTSCHRIFT**

(45) Veröffentlichungstag der Patentschrift: **19.08.92**

(51) Int. Cl.5: **C07D 239/50**, C07F 9/547, C07D 239/48, C07D 401/04, C07D 403/04, A01N 43/54, A01N 57/08, A01N 47/18, A01N 47/36, //C07D239/47

(21) Anmeldenummer: **87810262.3**

(22) Anmeldetag: **24.04.87**

Die Akte enthält technische Angaben, die nach dem Eingang der Anmeldung eingereicht wurden und die nicht in dieser Patentschrift enthalten sind.

# (54) Substituierte Pyrimidine.

(30) Priorität: **30.04.86 CH 1772/86**

(43) Veröffentlichungstag der Anmeldung:
**04.11.87 Patentblatt 87/45**

(45) Bekanntmachung des Hinweises auf die Patenterteilung:
**19.08.92 Patentblatt 92/34**

(84) Benannte Vertragsstaaten:
**AT BE CH DE ES FR GB GR IT LI NL**

(56) Entgegenhaltungen:
**DD-A- 157 192**
**DE-A- 2 738 653**
**DE-A- 3 205 638**
**FR-A- 2 206 909**
**US-A- 3 178 432**

**CHEMICAL ABSTRACTS, Band 59, Nr. 9, 28. Oktober 1963, Spalte 10038 b-d, Columbus, Ohio, US; D.E. O'BRIEN et al.: "Pyrimidines XI. Structural variations of 2,4-diamino-6-(haloanilino)-5-nitrosopyrimidines"**

**ZEITSCHRIFT FÜR CHEMIE, Band 21, Nr. 3, 1981, Seite 101; H. KRISTEN et al.: "Morpholinopyrimidines"**

(73) Patentinhaber: **CIBA-GEIGY AG**
**Klybeckstrasse 141**
**CH-4002 Basel(CH)**

(72) Erfinder: **Kristinsson, Haukur**
**Leimenstrasse 30**
**CH-4051 Basel(CH)**
Erfinder: **Kristiansen, Odd**
**Delligrabenstrasse 7**
**CH-4313 Möhlin(CH)**

**EP 0 244 360 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die vorliegende Erfindung betrifft neue, stabile substituierte 2,4-Diamino-5-cyano-Pyrimidine, ihre Salze und Zwischenprodukte, Verfahren zu ihrer Herstellung und ihre Verwendung zur Bekämpfung von Schadinsekten und Ektoparasiten.

Die neuen, stabilen erfindungsgemässen Verbindungen haben die allgemeine Formel I

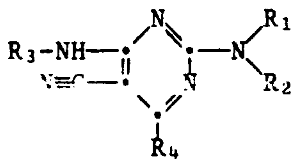

(I),

worin

| | |
|---|---|
| $R_1$ | Wasserstoff, $C_1$-$C_6$-Alkyl, $C_2$-$C_6$-Alkenyl oder $C_2$-$C_6$-Alkinyl; |
| $R_2$ | Wasserstoff, $C_1$-$C_{10}$-Alkyl oder $C_3$-$C_6$-Cycloakyl; oder |
| $R_1$ und $R_2$ | zusammen einen der Reste $-(CH_2)_3-$, $-(CH_2)_4-$ oder $-(CH_2)_5-$; |
| $R_3$ | Wasserstoff oder einen der Reste $-CO-R_5$ oder $-SO_2-R_6$ ; |
| $R_4$ | einen der Reste $-NH_2$, $-NH-CO-R_5$, $-NH-SO_2-R_6$ , 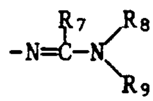 oder $-NH-CH=N-R_{10}$ ; |
| $R_5$ | Wasserstoff, $C_1$-$C_{12}$-Alkyl, $C_3$-$C_6$-Cycloalkyl, $C_1$-$C_6$-Alkoxy, perhalogeniertes $C_1$-$C_3$-Alkyl oder den Rest 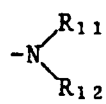 ; |
| $R_6$ | $C_1$-$C_6$-Alkyl; |
| $R_7$ | Wasserstoff oder $C_1$-$C_6$-Alkyl; |
| $R_8$ und $R_9$ | unabhängig voneinander Wasserstoff oder $C_1$-$C_6$-Alkyl oder zusammen einen der Reste $-(CH_2)_3-$, $-(CH_2)_4-$ oder $-(CH_2)_5-$; |
| $R_{10}$ | einer der Reste $-SO_2-R_{13}$ oder 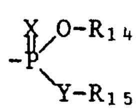 ; |
| $R_{11}$ und $R_{12}$ | unabhängig voneinander Wasserstoff oder $C_1$-$C_6$-Alkyl oder zusammen einen der Reste $-(CH_2)_3-$, $-(CH_2)_4-$ oder $-(CH_2)_5-$; |
| $R_{13}$ | $C_1$-$C_{10}$-Alkyl, mit bis zu 10 Halogenatomen substituiertes $C_1$-$C_{10}$-Alkyl oder $C_3$-$C_6$-Cycloalkyl; |
| $R_{14}$ und $R_{15}$ | unabhängig voneinander $C_1$-$C_{10}$-Alkyl; |
| X und Y | unabhängig voneinander Sauerstoff oder Schwefel bedeuten, sowie deren Salze. |

Unter Halogenatomen und Halogen-Substituenten im Rahmen der Erfindung sind Fluor, Chlor, Brom oder Jod, vorzugsweise Fluor und Chlor, zu verstehen.

Wegen ihrer vorteilhaften Wirkung sind solche Verbindungen der Formel I bevorzugt, worin

$R_1$ Wasserstoff oder $C_1$-$C_4$-Alkyl;
$R_2$ $C_1$-$C_4$-Alkyl oder $C_3$-$C_6$-Cycloalkyl oder
$R_1$ und $R_2$ zusammen einen der Reste $-(CH_2)_4-$ oder $-(CH_2)_5-$;
$R_3$ Wasserstoff;
$R_4$ einen der Reste $-NH_2$ oder $-NH-CO-R_5$; und
$R_5$ $C_1$-$C_4$-Alkyl oder perhalogeniertes $C_1$-$C_3$-Alkyl bedeuten, und deren Salze, sowie solche Verbindungen der Formel I, worin
$R_1$ Wasserstoff, Methyl oder Aethyl;
$R_2$ $C_1$-$C_4$-Alkyl oder Cyclopropyl;
$R_3$ Wasserstoff;
$R_4$ einen der Reste $-NH_2$ oder $-NH-CO-R_5$;
$R_5$ $C_1$-$C_3$-Alkyl bedeuten, sowie deren Salze.

Aufgrund ihrer hohen pestiziden Aktivität sind insbesondere Verbindungen der Formel I hervorzuheben, worin
$R_1$ Wasserstoff, Methyl oder Aethyl;
$R_2$ Cyclopropyl;
$R_3$ Wasserstoff; und
$R_4$ einen der Reste $-NH_2$ oder $-NH-CO-C_2H_5$ bedeuten, sowie deren Salze.

Unter Salzen der Verbindungen der Formel I sind die physiologisch unbedenklichen Additionssalze anorganischer und organischer Säuren zu verstehen. Beispiele anorganischer Säuren sind Chlorwasserstoffsäure, Bromwasserstoffsäure, Jodwasserstoffsäure, Schwefelsäure, Phosphorsäure, phosphorige Säure, Salpetersäure. Beispiele organischer Säuren sind Trifluoressigsäure, Trichloressigsäure, Ameisensäure, Oxalsäure, Bernsteinsäure, Maleinsäure, Milchsäure, Glycolsäure, Aconitsäure, Zitronensäure, Benzoesäure, Benzolsulfonsäure, Methansulfonsäure.

Die Verbindungen der Formel I können hergestellt werden, indem man
a) eine Verbindung der Formel II

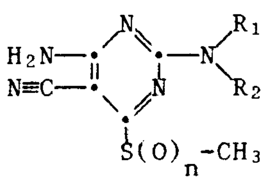

(II),

mit Ammoniak oder
b) die Verbindung der Formel III

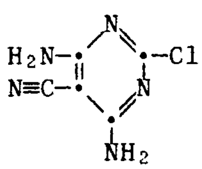

(III)

mit einem Amin der Formel (IV)

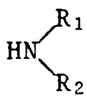

(IV)

umsetzt und die jeweils entstandene Verbindung der Formel (I), worin $R_3$ Wasserstoff und $R_4$ den Rest $-NH_2$ bedeuten (Verbindung Ia)

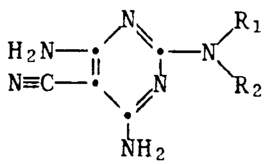 (IIa),

gegebenenfalls in an sich bekannter Weise in eine Verbindung der Formel I überführt, worin $R_3$ die Bedeutungen -CO-$R_5$ oder -$SO_2$-$R_6$ hat und/oder $R_4$ die Bedeutungen -NH-CO-$R_5$, -NH-$SO_2$- $R_6$,

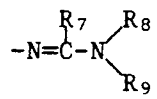

oder oder -NH = CH + N-$R_{10}$ hat, wobei $R_1$ bis $R_{15}$, X und Y die oben angegebenen Bedeutungen haben sowie n eine Zahl 0, 1 oder 2 bedeutet, und dass man erwünschtenfalls eine erhaltene Verbindung der Formel I in eines ihrer Salze überführt.

In Rahmen der vorstehenden Verfahrensvarianten a) erfolgt die Umsetzung einer Sulfonylverbindung (n = 2) der Formel II mit Ammoniak allgemein bei einer Temperatur von etwa 10 bis 100 ° C in organischen Lösungsmitteln, wie z.B. Acetonitril, Tetrahydrofuran, Dioxan oder Lösungsmittel-Wasser-Gemischen. Die gleichen Umsetzungsbedingungen gelten für die Verfahrensvariante b), bei der das 2-Chlor-4,6-di-amino-5-cyano-pyrimidin der Formel III mit einem Amin der Formel IV reagiert. Setzt man bei der Verfahrensvarianten a) Methylthioverbindungen (n = 0) der Formel II als Ausgangsprodukte ein, dann werden diese bevorzugt im Autoklaven unter erhöhtem Druck mit Ammoniak umgesetzt, und zwar bei einer Temperatur von 100 bis 170 ° C, zumeist bei etwa 160 ° C.

Die erwähnte Umwandlung einer Verbindung der Formel I, worin $R_4$ -$NH_2$ bedeutet, in eine solche, worin $R_4$ eine andere der erfindungsgemäss angegebenen Bedeutungen hat, kann durch Umsetzung der in 6-Stellung am Pyrimidinring befindlichen Aminogruppe mit entsprechenden an sich bekannten Umsetzungsteilnehmern erfolgen. So kann man z.B. ein 6-Aminopyrimidin der Formel I mit Verbindungen vom Typ Hal-CO-$R_5$, Hal-$SO_2$-$R_6$, ($R_5$-CO)$_2$O oder $R_6$O-CH = N-$R_{10}$ umsetzen, um zu in 6-Stellung entsprechend substituierte Verbindungen der Formel I zu gelangen, wobei Hal ein Halogenatom, vorzugsweise Chlor, darstellt und $R_5$, $R_6$ und $R_{10}$ die vorstehend angegebenen Bedeutungen haben. Wenn eine Verbindung der Formel I hergestellt werden soll, worin $R_4$ den Rest

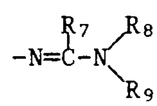

bedeutet, wird die 6-$NH_2$-Gruppe z.B. mit einem Acetal der Formel

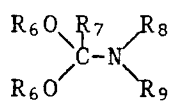

umgesetzt, wobei $R_6$, $R_7$, $R_8$ und $R_9$ die vorstehend angegebenen Bedeutungen haben. In analoger Weise und mit entsprechenden Umsetzungsteilnehmern kann man aus 4-Aminopyrimidinen der Formel I, worin $R_3$ Wasserstoff bedeutet, Verbindungen der Formel I herstellen, worin $R_3$ einen der Reste -CO-$R_5$ oder -$SO_2$-$R_6$ darstellt. Die vorstehend beschriebenen Acylierungen werden unter normalem Druck in inerten Lösungs- oder Verdünnungsmitteln in Gegenwart einer Base bei Temperaturen von 0 bis 120 ° C, vorzugsweise 40 bis 80 ° C, durchgeführt. Als Lösungs- und Verdünnungsmittel kommen beispielsweise in Frage:
Alkane wie n-Pentan sowie seine Homologen einschliesslich der Isomeren bis zum n-Heptadecan; Aether wie Diäthyläther, Dipropyläther, Dibutyläther, Dimethoxyäthan, Dioxan oder Tetrahydrofuran; Chlorierte Kohlenwasserstoffe, wie Chloroform Methylenchlorid, Tetrachlorkohlenstoff oder Chlorbenzol; aromatische Kohlenwasserstoffe wie Benzol, Toluol oder Xylole. Darüberhinaus können weitere inerte Lösungs- und Verdünnungsmittel bei dem Verfahren Anwendung finden. Als gegebenenfalls bei diesen Umsetzungen zu

verwendende Basen eignen sich beispielsweise Alkylamine, wie Triäthylamin oder Diisopropyläthylamin, sowie ferner Pyridin oder N-Methylpyrrolidon.

Die Ausgangsverbindungen der Formel II sind neu und bilden ebenfalls einen Gegenstand der vorliegenden Erfindung, insbesonders weil auch sie gute pestizide, speziell insektizide, Eigenschaften besitzen. Sie können wie folgt hergestellt werden:

a) So kann man z.B. zu einer Verbindung der Formel II, worin n = 0 ist, wie nachstehend schematisch angegeben, folgendermassen gelangen:

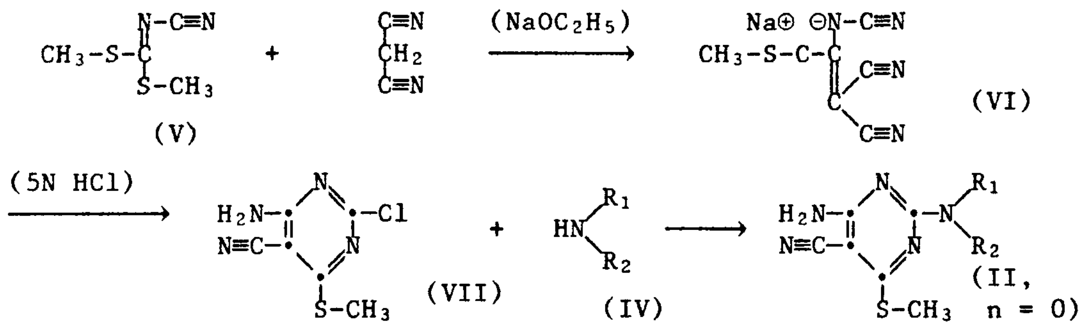

(V) (VI) (VII) (IV) (II, n = 0)

Der für die obige Arbeitsweise als Ausgangsprodukt verwendete N-Cyano-dithiocarbonimidsäuredimethylester der Formel V und das durch Umsetzung mit Malonitril entstehende Salz der Formel VI sind bekannt (vgl. Rec.Trav. Ch. 90/1971, 463; J.Chem.Soc., Chem. Comm. 1974, 350). Aus dem Salz VI kann man durch Ringschluss mit 5N HCl und anschliessende Umsetzung des gebildeten Pyrimidins der Formel VII mit einen entsprechenden Amin der Formel IV eine Verbindung der Formel II erhalten, in welcher n = 0 ist.

b) Weiterhin kann man Verbindungen der Formel II, worin n = 0 ist, wie folgt erhalten:

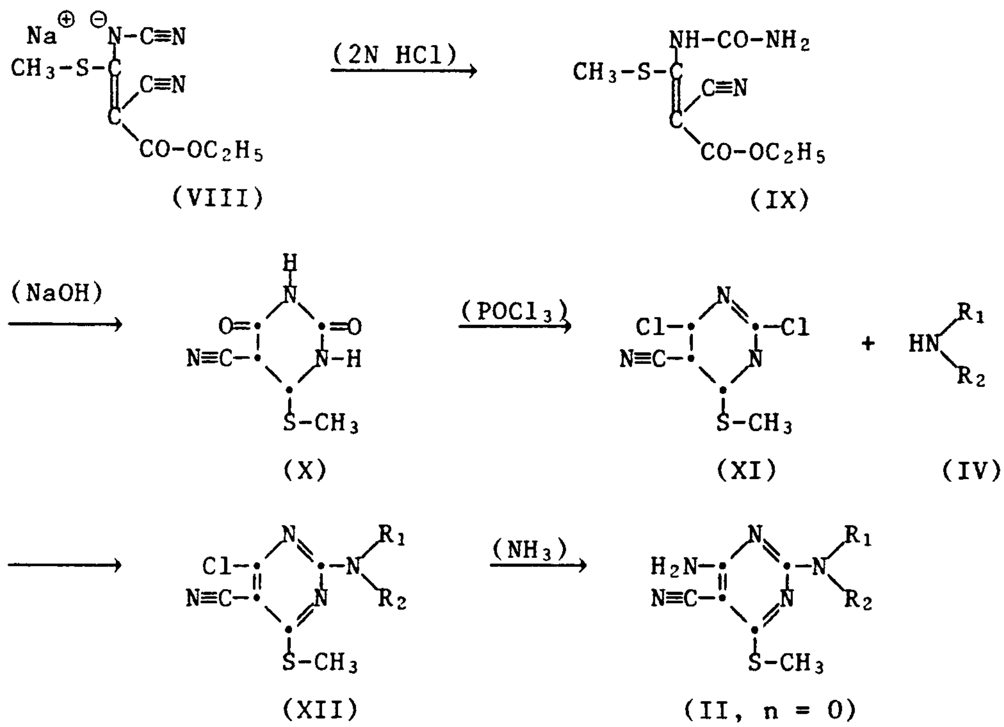

(VIII) (IX) (X) (XI) (IV) (XII) (II, n = 0)

Das im obigen Reaktionsschema angegebene Natriumsalz der Formel VIII, der Harnstoff der Formel IX sowie das Uracil der Formel X sind bekannt (vgl. J.Chem.Soc., Chem. Comm. 1974, 350; Helv. Chim. Act. 1985, 1155). Die Herstellung der neuen substituierten Pyrimidine der Formeln XI und XII erfolgt in an sich bekannter Weise. Durch Umsetzung von XII mit Ammoniak wird eine Verbindung der Formel II

erhalten, worin n = 0 ist.

c) Ausgangsverbindungen der Formel II, worin n = 1 oder 2 ist, können in an sich bekannter Weise durch Oxydation von Verbindungen der Formel II, worin n = 0 ist, hergestellt werden (vgl. "The Chemistry of Heterocyclic Compounds", Vol. 16: Pyrimidines, Intersc. Publ. Inc., N.Y. 1959).

Die Ausgangsverbindung der Formel III, d.h. das 2-Chlor-4,6-di-amino-5-cyano-pyrimidin, ist aus Chem. Ber. 1968, 1244, bekannt, kann aber gemäss folgendem Schema aus der obigen Verbindung der Formel VII durch Oxydation zu Sulfon und Austausch von $-SO_2CH_3$ durch $-NH_2$ mittels Ammoniak hergestellt werden:

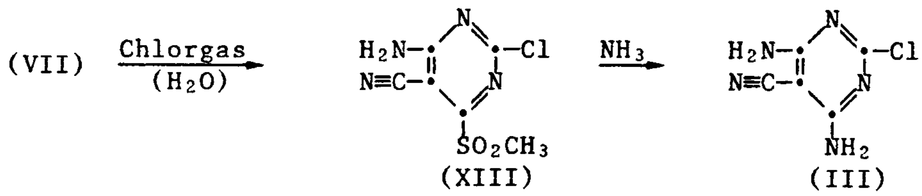


(XIII) (III)

Pestizid, insbesondere insektizid, wirksame, 2,4,6-Triamino-5-nitropyrimidine, deren Aminogruppen gegebenenfalls substituiert sein können, sind bereits aus der europäischen Patentanmeldung 0084.758 bekannt. Gegenüber diesen bekannten Verbindungen unterscheiden sich die erfindungsgemässen Wirkstoffe der Formeln I und II strukturell im wesentlichen durch das Vorliegen der Cyano-Gruppe in 5-Stellung.

Ueberraschenderweise wurde gefunden, dass die vorliegenden Verbindungen der Formeln I und II sowie ihre Salze bei guter Pflanzenverträglichkeit und geringer Warmblütertoxizität ausgezeichnete Wirksamkeit als Schädlingsbekämpfungsmittel aufweisen. Sie eignen sich vor allem zur Bekämpfung von Pflanzen und Tiere befallenden Insekten und Vertretern der Ordnung Akarina.

Insbesondere eignen sich die Verbindungen der Formeln I und II zur Bekämpfung von Insekten der Ordnungen; Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera, sowie von Vertretern der Ordnung Akarina der Familien: Ixodidae, Argasidae, Tetranychidae und Dermanyssidae.

Neben ihrer Wirkung gegenüber Fliegen, wie z.B. Musca domestica, und Mückenlarven können die erfindungsgemässen Wirkstoffe auch zur Bekämpfung von pflanzenschädigenden Frassinsekten in Zier- und Nutzpflanzungen, insbesondere in Baumwollkulturen (z.B. Spodoptera littoralis und Heliothis virescens), sowie in Obst- und Gemüsekulturen (z.B. Plutella xylostella, Laspeyresia pomonella, Leptinotarsa decemlineata und Epilachna varivestis) eingesetzt werden. Die erfindungsgemässen Verbindungen der Formel I besitzen auch gute Wirksamkeit gegen Reis-Schädlinge. Die Verbindungen der Formel I zeichnen sich ferner durch eine ausgeprägte larvizide Wirkung gegen Insekten, insbesondere Larven von fressenden Schadinsekten, aus. Werden die Wirkstoff-Verbindungen von adulten Insekten-Stadien mit dem Futter aufgenommen, so ist in vielen Fällen, insbesondere bei Coleopteren, wie z.B. Anthonomus grandis, eine verminderte Ei-Ablage und/oder reduzierte Schlupfrate festzustellen.

Die Wirkstoffe der Formeln I und II können darüberhinaus mit sehr guten Resultaten zur Bekämpfung von Ektoparasiten, wie Lucilia sericata, an Haus- und Nutztieren eingesetzt werden, z.B. durch Tier-, Stall- und Weidebehandlung.

Bei der Behandlung von Weidetieren mit den erfindungsgemässen Wirkstoffen, z.B. mittels Viehbädern, Aufguss-Methoden oder Sprühgängen, wird durch die überraschende Adhäsionswirkung der Aktivsubstanzen ein lang anhaltender toxischer Effekt gegen Ektoparasiten, wie beispielsweise schädlichen Dipteren, auf der Haut und dem Fell der Tiere erzielt. Ein frühzeitiges Aus- oder Abwaschen der auf die Oberfläche der Nutztiere applizierten Wirkstoffe durch ablaufendes Regenwasser kann somit verhindert werden.

Ein besonderer Vorteil der erfindungsgemässen Wirkstoff-Verbindungen besteht in ihrer oralen Verabreichung an Nutztiere. Bei diesem Anwendungsverfahren entfalten die Wirkstoffe vor allem in den aus dem Verdauungstrakt ausgeschiedenen Fäkalien eine nachhaltige und langandauernde insektizide Aktivität. Dadurch kann der Befall mit schädlichen Insekten, insbesonders Dipteren, bereits vor dem Auftreten der Schädlinge in der Umgebung der Tiere, wie Stallungen, Gehegen und Weiden, verhindert werden, weil die aus den abgelegten Eiern schlüpfenden Dipterenlarven sofort abgetötet werden. Bei dieser speziellen Anwendung ist besonders bedeutsam, dass sich die Verbindungen aufgrund ihrer strukturellen Eigenschaften gegenüber Warmblütern physiologisch indifferent verhalten. Diese Methode der gezielten Bekämpfung der Proliferation der Insekten ist bedeutend effizienter und gleichzeitig ökonomischer als die üblichen grossflächigen Desinfektionen von Stallungen und Gehegen.

Die gute pestizide Wirkung der erfindungsgemässen Verbindungen der Formeln I und II entspricht einer

Abtötungsrate (Mortalität) von mindestens 50-60 % der erwähnten Schädlinge.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die erfindungsgemässen Verbindungen werden in unveränderter Form oder vorzugsweise zusammen mit den in der Formulierungstechnik üblichen Hilfsmitteln eingesetzt und können daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff der Formel I oder II, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes oder der Kombinationen dieser Wirkstoffe mit andern Insektiziden oder Akariziden nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäure-methyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B . das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten.

Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3

bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstecknik gebräuchlichen Tenside sind u.a. in folgenden Publikationen beschrieben:
"Mc Cutcheon's Detergents and Emulsifiers Annual"
MC Publishing Corp., Ridgewood, New Jersey, 1979;
Dr. Helmut Stache "Tensid Taschenbuch", Carl Hanser Verlag München/Wien 1981.

Die pestiziden Zubereitungen enthalten in der Regel 0,1 bis 99 %, insbesondere 0,1 bis 95 %, des erfindungsgemässen Wirkstoffs oder Kombinationen dieser Wirkstoffe mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1

a) Herstellung der Ausgangsverbindung 2-Cyclopropylamino-4-amino-5-cyano-6-methylthio-pyrimidin:

i) Zu einer Lösung von 93 g 2-Cyano-3-cyanoamino-3-methylthio-acrylnitril-Natriumsalz in 425 ml Wasser werden 650 ml konzentrierte Salzsäure unter Kühlung mittels Eiswasser zugetropft. Dann wird der Ansatz während ca. 12 Stunden bei Raumtemperatur gerührt. Der entstandene Niederschlag wird abfiltriert und mit wässriger Sodalösung behandelt. Das so erhaltene 2-Chlor-4-amino-5-cyano-6-methylthio-pyrimidin (Smp.: 268°C) wird in einer Menge von 20 g mit 250 ml Acetonitril zu einer Suspension verrührt, in welche unter kräftigem Rühren 11,4 g Cyclopropylamin bei Rückflusstemperatur eingetropft werden. Der Ansatz wird etwa 12 Stunden weiter gerührt und dann mit Wasser versetzt. Der gebildete Niederschlag wird abfiltriert. Man erhält so die Titelverbindung der Formel

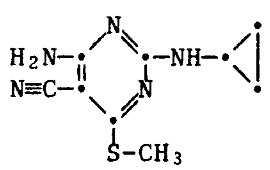

mit einem Smp. von 215-218°C (Verbindung Nr. 3.1.).

ii) Zu einem Gemisch aus 18,3 g 5-Cyano-6-methylthiouracil und 80 ml Phosphoroxychlorid werden 20 g N,N-Diäthylanilin langsam zugetropft. Der Ansatz wird dann während 1,5 Stunden am Rückfluss erhitzt und nachfolgend eingedampft. Der Rückstand wird mit 200 ml Eiswasser versetzt, verrührt, abfiltriert und mit Wasser gewaschen. Nach dem Trocknen wird das Rohprodukt an Kieselgel chromatographiert (Toluol/Chloroform/Essigester 6:3:1). 22 g des so erhaltenen 2,4-Dichlor-5-cyano-6-methylthio-pyrimidins (Smp. 118-120°C) werden in 200 ml Acetonitril gelöst, und bei -10°C wird eine Lösung von 11,4 g Cyclopropylamin in 40 ml Acetonitril zugetropft. Das Reaktionsgemisch wird bei Raumtemperatur während 2 Stunden gerührt und dann auf 2 ℓ Eiswasser gegossen. Der gebildete Rückstand wird

abgesaugt. Das nach obiger Arbeitsweise erhaltene 2-Cyclopropylamino-4-chlor-5-cyano-6-methylthiopyrimidin der Formel

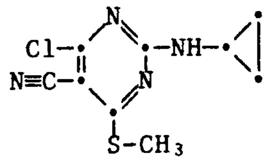

mit einem Smp. von 139-141 ° C wird in einer Menge von 26,1 g mit 100 ml Acetonitril verrührt. Es werden 300 ml 30 %-iges wässriges Ammoniak zu der so entstandenen Suspension hinzugefügt, die dann etwa 10 Stunden bei Raumtemperatur und anschliessend 5 Stunden am Rückflusse gerührt wird. Der nach dem Abkühlen des Ansatzes entstandene feste Rückstand wird abgesaugt und mit Wasser gewaschen. Nach dem Umkristallisieren aus 150 ml Methylcellosalve wird die Titelverbindung mit der unter i) angegebenen Formel vom Smp. 215-218 ° C erhalten.

b) Herstellung von 2-Cyclopropylamino-4,6-diamion-5-cyano-pyrimidin:

Es werden 33,7 g des gemäss a) hergestellten 2-Cyclopropylamino-4-amino-5-cyano-6-methylthiopyrimidins im Autoklaven bei 150 ° C während 15-20 Stunden mit 150 g Ammoniak behandelt. Das gebildete Reaktionsprodukt wird mehrmals mit Wasser verrührt und dann abfiltriert. Man erhält auf diese Weise die Titelverbindung der Formel

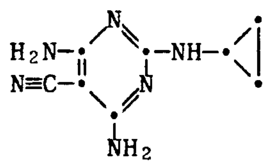

mit einem Smp. von 249-251 ° C (Verbindung Nr. 1.1).

Beispiel 2

a) Herstellung der Ausgangsverbindung 2-Diäthylamino-4-amino-5-cyano-6-methylsulfonyl-pyrimidin:

Es werden 47,5 g 2-Diäthylamino-4-amino-5-cyano-6-methylthio-pyrimidin in 650 ml Methylenchlorid vorgelegt. Zu dem Ansatz werden 88,7 g 3-Chlorperbenzoesäure ohne Kühluing hinzugefügt. Das Reaktionsgemisch wird während 2 Stunden gerührt und dann filtriert. Das Filtrat wird eingedampft, der Rückstand mit Diäthyläther aufgeschlämmt und abgesaugt. Man erhält die Titelverbindung der Formel

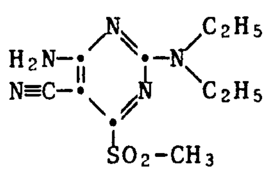

mit einem Smp. von 170-172 ° C (Verbindung Nr. 3.2).

b) Herstellung von 2-Diäthylamino-4,6-diamino-cyano-pyrimidin:

Zu einem aus 300 ml 30 %-igesm wässrigen Ammoniak und 100 ml Acetonitril bestehenden Gemisch werden bei Raumtemperatur 26,9 g des gemäss a) hergestellten 2-Diäthylamino-4-amino-5-cyano-6-methylsulfonyl-pyrimidins eingetragen. Der Ansatz wird dann etwa 12 Stunden am Rückfluss gehalten, abgekühlt und von dem gebildeten Niederschlag abfiltriert. Der Niederschlag wird mit Wasser behandelt und abgesaugt. Man erhält so die Titelverbindung der Formel

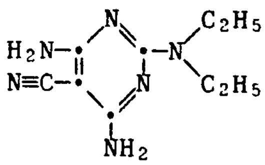

mit einem Smp. von 222-224° C (Verbindung Nr. 1.2).

Beispiel 3

Herstellung von 2-Diäthylamino-4-amino-5-cyano-6-isobutyrylamino-pyrimidin:

Zu einer Lösung von 10,3 g 2-Diäthylamino-4,6-diamino-5-cyano-pyrimidin(hergestellt nach Beispiel 2) in 90 ml Tetrahydrofuran werden 7 g Triäthylamin hinzugesetzt. In diese Lösung werden bei 60° C 8,5 g Isobuttersäureanhydrid eingetropft. Das Reaktionsgemisch wird während 48 Stunden am Rückfluss erhitzt, eingedampft und der gebildete Rückstand mit Diäthyläther gewaschen. Die so erhaltene Titelverbindung der Formel

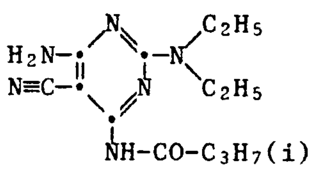

hat einem Smp. von 151-152° C (Verbindung Nr. 1.3).

Beispiel 4:

Herstellung von N,N-Dimethyl-N′-(2-cyanopropylamino-4-amino-5-cyano-pyrimidinyl-6)-formamidin:

Es werden 3,7 g Dimethylformamiddiäthylacetal zu einer Lösung von 3,8 g 2-Cyclopropylamino-4,6-diamino-5-cyanopyrimidin in 100 ml Dioxan bei 45°-50° C zugetropft. Das Reaktionsgemisch wird während 8 Stunden bei 45 bis 50° C gerührt. Nach dem Abdestillieren des Lösungsmittels und Umkristallisieren des Rohproduktes aus Aethanol wird die Titelverbindung der Formel

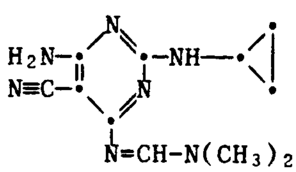

mit einem Smp. von 192-195° C erhalten (Verbindung Nr. 1.4).

Beispiel 5

Herstellung der Ausgangsverbindung 2-Chlor-4,6-diamino-5-cyano-pyrimidin:

Eine Suspension von 50 g feinpulverisiertem 2-Chlor-4-amino-5-cyano-6-methylthiopyrimidin in 750 ml Dioxan und 250 ml Wasser wird bei 0° C vorgelegt. Dann wird in diese Suspension unter Rühren und ohne Kühlung ein kräftiger Chlorstrom eingeleitet. Nach ca. 20 Minuten resultiert eine klare Lösung. Nach weiteren 30 Minuten wird das Einleiten des Chlors beendet und der Ansatz wird für 1/2 Stunde ausgerührt. Das Reaktionsgemisch wird eingedampft, der Rückstand mit 600 ml Eiswasser aufgeschlämmt und dann abfiltriert. Das als Filterrückstand erhaltene 2-Chlor-4-amino-5-cyano-6-methylsulfonyl-pyrimidin (Smp.: 253° C Zers.) wird in einer Menge von 46,4 g mit 25 g 30%-iger wässriger Ammoniaklösung und 1000 ml

Acetonitril bei Raumtemperatur während 2 Stunden gerührt. Nach dem Eindampfen wird der Ansatz mit Wasser aufgeschlämmt und abfiltriert.
Man erhält so die Titelverbindung der Formel

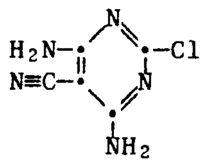

mit einem Smp. von >260 ° C (Verbindung Nr. 5.1).

Analog den vorstehend aufgezeigten Arbeitsweisen werden auch die folgenden Verbindungen der Formel I hergestellt:

| Verb. Nr. | $R_1$ | $R_2$ | $R_3$ | $R_4$ | Smp. [°C] |
|---|---|---|---|---|---|
| 1.5 | H | $-C_3H_7(i)$ | H | $-NH_2$ | 230-232 |
| 1.6 | H | $-CH(CH_3)-C_2H_5$ | H | $-NH_2$ | 195-197 |
| 1.7 | $-CH_3$ | $-CH_3$ | H | $-NH_2$ | > 260 |
| 1.8 | $-C_3H_7(n)$ | $-C_3H_7(n)$ | H | $-NH_2$ | 204-206 |
| 1.9 | $-C_4H_9(n)$ | $-C_4H_9(n)$ | H | $-NH_2$ | 182-185 |
| 1.10 | $-CH_3$ | $-C_2H_5$ | H | $-NH_2$ | 250-251 |
| 1.11 | $-(CH_2)_4-$ | | H | $-NH_2$ | > 260 |
| 1.12 | $-(CH_2)_5$ | | H | $-NH_2$ | 234-236 |
| 1.13 | H | $-C(CH_3)_3$ | H | $-NH_2$ | 180-183 |
| 1.14 | $-CH_3$ | $-C_4H_9(n)$ | H | $-NH_2$ | 194-196 |
| 1.15 | H | 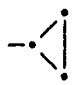 | H | $-NH-CO-CH(CH_3)_2$ | 215-218 |
| 1.16 | H | 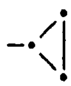 | H | $-NH-CO-C_2H_5$ | 180-181 |
| 1.17 | H | 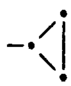 | H | $-NH-CO-CH_3$ | 203-205 |
| 1.18 | $-C_2H_5$ | $-C_2H_5$ | H | $-NH-CO-C_2H_5$ | 158-160 |
| 1.19 | $-C_2H_5$ | $-C_2H_5$ | H | $-NH-CO-C(CH_3)_3$ | 113-115 |
| 1.20 | $-C_2H_5$ | $-C_2H_5$ | H | $-NH-CO-CH_3$ | 168-171 |
| 1.21 | $-C_2H_5$ | $-C_2H_5$ | H | $-NH-CO-C_3H_7(n)$ | 149-150 |
| 1.22 | H | 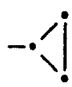 | H | $-NH-CO-C(CH_3)_3$ | 110-113 |
| 1.23 | $-C_2H_5$ | $-C_2H_5$ | H | $-NH-CO-C_4H_9(n)$ | 119-120 |
| 1.24 | H | 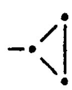 | H | $-NH-CO-C_3H_7(n)$ | 175-176 |
| 1.25 | H | 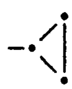 | H | $-NH-CO-C_4H_9(n)$ | 187-188 |
| 1.26 | $-C_2H_5$ | $-C_2H_5$ | H | $-NH-CO-CF_3$ | 190-191 |

| Verb. Nr. | $R_1$ | $R_2$ | $R_3$ | $R_4$ | Smp. [°C] |
|---|---|---|---|---|---|
| 1.27 | H | 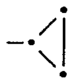 | H | $-NH-CO-CF_3$ | 268 |
| 1.28 | $-CH_3$ | $-C_4H_9(n)$ | H | $-NH-CO-CH(CH_3)_2$ | 139-140 |
| 1.29 | $-CH_3$ | $-C_2H_5$ | H | $-NH-CO-CH(CH_3)_2$ | 146-149 |
| 1.30 | H | 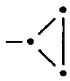 | H | $-NH-CO-CH(C_2H_5)_2$ | 196-197 |
| 1.31 | $-C_2H_5$ | $-C_2H_5$ | H | $-NH-CO-(CH_2)_4CH_3$ | 133-135 |
| 1.32 | H | 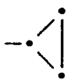 | H | $-NH-CO-(CH_2)_4CH_3$ | 170-173 |
| 1.33 | H | 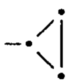 | H | $CH_3$<br>$-N=CH-N-CH_3$ | 192-195 |
| 1.34 | $-C_2H_5$ | $-C_2H_5$ | H | $CH_3$<br>$-N=CH-N-CH_3$ | 160-165 |
| 1.35 | H | 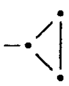 | H | $-N=CH-NH-SO_2CH_3$ | 203-205 |
| 1.36 | $-C_2H_5$ | $-C_2H_5$ | H | $-N=CH-NH-SO_2CH_3$ | 182-185 |
| 1.37 | $-C_2H_5$ | $-C_2H_5$ | H | O $OC_2H_5$<br>$-N=CH-NH-P$<br>$SC_3H_7(n)$ | 127-128 |
| 1.38 | $-CH_2-CH=CH_2$ | $-CH_3$ | H | $-NH_2$ | 199-201 |
| 1.39 | $-CH_2-CH=CH_2$ | H | H | $-NH_2$ | 191-193 |
| 1.40 | $-CH_2-C\equiv CH$ | H | H | $-NH_2$ | >260 |
| 1.41 | H | $-CH_2-$ 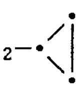 | H | $-NH_2$ | 228-229 |
| 1.42 | H | 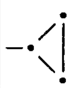 | $-CO-CH_3$ | $-NH-CO-CH_3$ | ~ 260 |
| 1.43 | $-C_2H_5$ | $-C_2H_5$ | $-CO-CH_3$ | $-NH-CO-CH_3$ | 263-265 |

Die folgenden Salze von Verbindungen der Formel I werden durch Umsetzung mit den jeweils bezeichneten Säuren hergestellt:

| Verb. Nr. | $R_1$ | $R_2$ | $NHR_3$ | $R_4$ | Säure | Smp. [°C] |
|---|---|---|---|---|---|---|
| 2.1 | H | 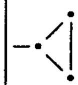 | $-NH_2$ | $-NH_2$ | HCl | 210 (Zers.) |
| 2.2 | H | 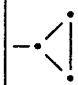 | $-NH_2$ | $-NH_2$ | $F_3C-COOH$ | 209-210 |
| 2.3 | H | 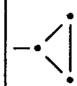 | $-NH_2$ | $-NH_2$ | $(COOH)_2$ | 198 (Zers.) |
| 2.4 | $-C_2H_5$ | $-C_2H_5$ | $-NH_2$ | $-NH_2$ | HCl | 167-169 |
| 2.5 | $-C_2H_5$ | $-C_2H_5$ | $-NH_2$ | $-NH_2$ | $F_3C-COOH$ | 175-178 |
| 2.6 | $-C_2H_5$ | $-C_2H_5$ | $-NH_2$ | $-NH_2$ | $(COOH)_2$ | 178-188 |
| 2.7 | H | 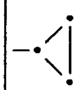 | $-NH_2$ | $-NH-CO-CH(CH_3)_2$ | HCl | ~140 |
| 2.8 | H | 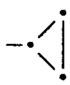 | $-NH_2$ | $-NH-CO-CH(CH_3)_2$ | $CF_3COOH$ | 150-153 |
| 2.9 | H | 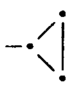 | $-NH_2$ | $-NH_2$ | $H_3PO_4$ | 208 |
| 2.10 | H | 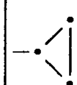 | $-NH_2$ | $-NH_2$ | $H_2SO_4$ | 188 |
| 2.11 | $-C_2H_5$ | $-C_2H_5$ | $-NH_2$ | $-NH_2$ | $H_2SO_4$ | 199-201 |
| 2.12 | $-C_2H_5$ | $-C_2H_5$ | $-NH_2$ | $-NH_2$ | $H_3PO_4$ | 185 |
| 2.13 | $-C_2H_5$ | $-C_2H_5$ | $-NH_2$ | $-NH_2$ | $CH_3SO_3H$ | 205-207 |
| 2.14 | H | 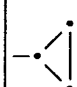 | $-NH_2$ | $-NH_2$ | $CH_3SO_3H$ | 250 |
| 2.15 | $-C_2H_5$ | $-C_2H_5$ | $-NH_2$ | $-NH_2$ | Maleinsäure | 150 |
| 2.16 | H | 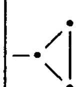 | $-NH_2$ | $-NH_2$ | Maleinsäure | 185 |
| 2.17 | $-C_2H_5$ | $-C_2H_5$ | $-NH_2$ | $-NH_2$ | Malonsäure | 216-220 |

| Verb. Nr. | $R_1$ | $R_2$ | $NHR_3$ | $R_4$ | Säure | Smp. [°C] |
|---|---|---|---|---|---|---|
| 2.18 | H | 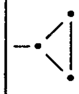 | $-NH_2$ | $-NH_2$ | $HNO_3$ | 163 |
| 2.19 | $-C_2H_5$ | $-C_2H_5$ | $-NH_2$ | $-NH_2$ | $HNO_3$ | 179 |
| 2.20 | H | 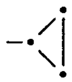 | $-NH_2$ | $-NH_2$ | Malonsäure | >230 |

Wie vorstehend angegeben sind auch die folgenden Verbindungen der Formel I herstellbar:

| $R_1$ | $R_2$ | $R_3$ | $R_4$ |
|---|---|---|---|
| H | $-CH_3$ | H | $-NH_2$ |
| H | $-C_2H_5$ | H | $-NH_2$ |
| H | $-C_3H_7(n)$ | H | $-NH_2$ |
| H | 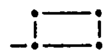 | H | $-NH_2$ |
| H | 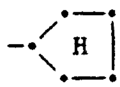 | H | $-NH_2$ |
| H | 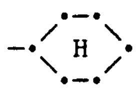 | H | $-NH_2$ |
| H | $-CH(C_2H_5)_2$ | H | $-NH_2$ |
| $-C_3H_7(i)$ | $-C_3H_7(i)$ | H | $-NH_2$ |
| H | 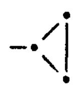 | H | 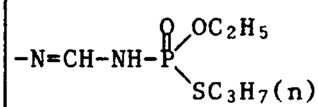 |
| H | 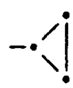 | H | $-NH-CO-O-C_2H_5$ |
| $-C_2H_5$ | $-C_2H_5$ | H | $-NH-CO-O-C_2H_5$ |
| $-C_2H_5$ | $-C_2H_5$ | H | $-NH-CO-CH_2Cl$ |
| H | 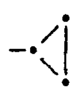 | H | $-NH-CO-C_2F_5$ |
| $-C_2H_5$ | $-C_2H_5$ | H | $-NH-CO-C_2F_5$ |
| H | 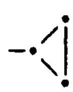 | H | $-NH-CO-C_3F_7(n)$ |
| $-C_2H_5$ | $-C_2H_5$ | H | $-NH-CO-C_3F_7(n)$ |
| H | 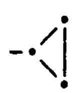 | $-CO-C_2H_5$ | $-NH-CO-C_2H_5$ |
| H | 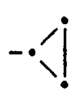 | $-CO-C_3H_7(n)$ | $-NH-CO-C_3H_7(n)$ |
| H | 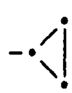 | $-CO-CH(CH_3)_2$ | $-NH-CO-CH(CH_3)_2$ |

| $R_1$ | $R_2$ | $R_3$ | $R_4$ |
|---|---|---|---|
| $-C_2H_5$ | $-C_2H_5$ | $-CO-C_2H_5$ | $-NH-CO-C_2H_5$ |
| $-C_2H_5$ | $-C_2H_5$ | $-CO-C_3H_7(n)$ | $-NH-CO-C_3H_7(n)$ |
| $-C_2H_5$ | $-C_2H_5$ | $-CO-CH(CH_3)_2$ | $-NH-CO-CH(CH_3)_2$ |
| H | 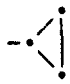 | $-CO-CF_3$ | $-NH-CO-CF_3$ |
| $-C_2H_5$ | $-C_2H_5$ | $-CO-CF_3$ | $-NH-CO-CF_3$ |
| H | 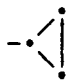 | $-CO-CH_3$ | $-NH-CO-CH(CH_3)_2$ |
| $-C_2H_5$ | $-C_2H_5$ | $-CO-C_2H_5$ | $-NH-CO-CH(CH_3)_2$ |

Wie vorstehend angegeben, werden die folgenden Zwischenprodukte der Formel II hergestellt:

| Verb. Nr. | $R_1$ | $R_2$ | $R_3$ | n | Smp. [°C] |
|---|---|---|---|---|---|
| 3.3 | H | $-CH_3$ | H | 0 | 270-274 |
| 3.4 | H | $-C_3H_7(i)$ | H | 0 | 171-173 |
| 3.5 | $-(CH_2)_4-$ | | H | 0 | 198-200 |
| 3.6 | $-(CH_2)_5-$ | | H | 0 | 204-206 |
| 3.7 | H | $-C(CH_3)_3$ | H | 0 | 194-196 |
| 3.8 | H | $-CH(CH_3)-C_2H_5$ | H | 0 | 143-145 |
| 3.9 | H | $-CH(C_2H_5)_2$ | H | 0 | 151-153 |
| 3.10 | $-CH_3$ | $-CH_3$ | H | 0 | 216-218 |
| 3.11 | $-C_2H_5$ | $-C_2H_5$ | H | 0 | 131 |
| 3.12 | $-CH_3$ | $-C_2H_5$ | H | 0 | 158-160 |
| 3.13 | $-CH_3$ | $-C_4H_9(n)$ | H | 0 | 158-160 |
| 3.14 | $-C_3H_7(n)$ | $-C_3H_7(n)$ | H | 0 | 160-162 |
| 3.15 | $-C_4H_9(n)$ | $-C_4H_9(n)$ | H | 0 | 120-122 |
| 3.16 | H | H | H | 0 | ~270 (Zers.) |
| 3.17 | H | 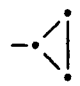 | H | 2 | 167-170 |
| 3.18 | $-CH_3$ | $-CH_3$ | H | 2 | 237-239 |
| 3.19 | $-C_3H_7(n)$ | $-C_3H_7(n)$ | H | 2 | 222-225 |
| 3.20 | $-C_4H_9(n)$ | $-C_4H_9(n)$ | H | 2 | 165-167 |
| 3.21 | $-CH_3$ | $-C_2H_5$ | H | 2 | 203-205 |
| 3.22 | $-(CH_2)_4-$ | | H | 2 | 235-237 |
| 3.23 | $-(CH_2)_5-$ | | H | 2 | 204-207 |
| 3.24 | $-CH_3$ | $-C_4H_9(n)$ | H | 2 | 190-191 |

Wie vorstehend angegeben, sind auch die folgenden Zwischenprodukte der Formel II herstellbar:

| R1 | R2 | R3 | n |
|---|---|---|---|
| H | $-C_2H_5$ | H | 0 |
| H | $-C_3H_7$(n) | H | 0 |
| H | $-C_3H_7$(i) | H | 0 |
| H | $-CH(C_2H_5)_2$ | H | 0 |
| H | 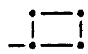 | H | 0 |
| H | 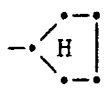 | H | 0 |
| H | 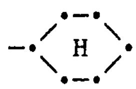 | H | 0 |
| H | $-CH(CH_3)-C_2H_5$ | H | 0 |
| $-CH_3$ | $-CH_3$ | H | 0 |
| $-C_3H_5$ | $-C_2H_5$ | H | 0 |
| H | $-CH_3$ | H | 2 |
| H | $-C_2H_5$ | H | 2 |
| H | $-C_3H_7$(n) | H | 2 |
| $-C_3H_7$(i) | $-C_3H_7$(i) | H | 2 |
| H | 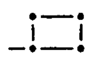 | H | 2 |
| H | 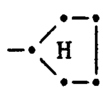 | H | 2 |
| H | 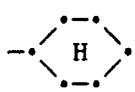 | H | 2 |
| H | $-CH(CH_3)-C_2H_5$ | H | 2 |
| H | $-CH(C_2H_5)_2$ | H | 2 |

Wie vorstehend angegeben, werden die folgenden Zwischenprodukte der Formel XI hergestellt:

| Verb. Nr. | $R_1$ | $R_2$ | Smp. [°C] |
|---|---|---|---|
| 4.1 | H | $-CH_3$ | 270-274 |
| 4.2 | H | $-C_3H_7(i)$ | 171-173 |
| 4.3 | H | 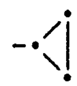 | 215-218 |
| 4.4 | H | $-CH(CH_3)-C_2H_5$ | 143-145 |
| 4.5 | H | $-CH(C_2H_5)_2$ | 151-153 |
| 4.6 | $-CH_3$ | $-CH_3$ | 216-218 |
| 4.7 | $-C_2H_5$ | $-C_2H_5$ | 131 |
| 4.8 | $-C_3H_7(n)$ | $-C_3H_7(n)$ | 160-162 |
| 4.9 | $-C_4H_9(n)$ | $-C_4H_9(n)$ | 120-122 |
| 4.10 | H | H | 270 (Zers.) |
| 4.11 | $-CH_3$ | $-C_2H_5$ | 158-160 |
| 4.12 | H | $-C(CH_3)_3$ | 194-196 |
| 4.13 | $-(CH_2)_4-$ | | 198-200 |
| 4.14 | $-(CH_2)_5-$ | | 204-206 |
| 4.15 | $-CH_3$ | $-C_4H_9(n)$ | 158-160 |

Wie vorstehend angegeben, sind auch die folgenden Zwischenprodukte der Formel XI herstellbar:

| $R_1$ | $R_2$ |
|---|---|
| H | $-C_2H_5$ |
| H | $-C_3H_7(n)$ |
| H | 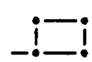 |
| H | 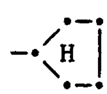 |
| H | 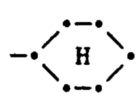 |
| $-C_3H_7(i)$ | $-C_3H_7(i)$ |

Beispiel 6: Formulierungen für Wirkstoffe gemäss den Beispielen 1 bis 3 resp. Kombinationen dieser Wirkstoffe mit andern Insektiziden oder Akariziden (% = Gewichtsprozent)

| 1. Spritzpulver | a) | b) | c) |
|---|---|---|---|
| Wirkstoff oder Wirkstoffkombination | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2. Emulsions-Konzentrat | |
|---|---|
| Wirkstoff oder Wirkstoffkombination | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 3. Stäubemittel | a) | b) |
|---|---|---|
| Wirkstoff oder Wirkstoffkombination | 5 % | 8 % |
| Talkum | 95 % | - |
| Kaolin | - | 92 % |

Man erhält anwendungsfertige Stäubemittel, indem der Wirkstoff oder die Wirkstoffkombination mit dem Träger vermischt und auf einer geeigneten Mühle vermahlen wird.

| 4. Extruder-Granulat | |
|---|---|
| Wirkstoff oder Wirkstoffkombination | 10 % |
| Na-Ligninsulfonat | 2 % |
| Carboxymethylcellulose | 1 % |
| Kaolin | 87 % |

Der Wirkstoff oder die Wirkstoffkombination wird mit den Zusatzstoffen vermischt, vermahlen und mit Wasser angefeuchtet. Dieses Gemisch wird extrudiert, granuliert und anschliessend im Luftstrom getrocknet.

| 5. Umhüllungs-Granulat | |
|---|---|
| Wirkstoff oder Wirkstoffkombination | 3 % |
| Polyäthylenglykol (MG 200) | 3 % |
| Kaolin | 94 % |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

| 6. Suspensions-Konzentrat | |
|---|---|
| Wirkstoff oder Wirkstoffkombination | 40 % |
| | Aethylenglykol 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

7. Aufguss-Lösung ("pour-on"-Zubereitung)

| | |
|---|---|
| Wirksubstanz | 30,00 g |
| Natrium-dioctylsulfosuccinat | 3,00 g |
| Benzylalkohol | 35,46 g |
| Aethylenglykol-monomethyläther | 35,46 g |
| | 103,92 g = 100 ml |

Die Wirksubstanz wird in dem grössten Teil des Gemisches der beiden Lösungsmittel unter kräftigem Rühren, gelöst. Anschliessend wird das Natrium-dioctylsulfosuccinat, eventuell unter Erwärmen, gelöst und schliesslich mit dem restlichen Lösungsmittelgemisch aufgefüllt.

Beispiel 7: Wirkung gegen Musca domestica:

Je 50 g frisch zubereitetes Nährsubstrat für Maden werden in Becher eingewogen. Von einer 1 Gew.-%igen acetonischen Lösung des betreffenden Wirkstoffes wird eine bestimmte Menge auf das in den Bechern befindliche Nährsubstrat pipettiert, so dass sich eine Wirkstoffkonzentration von 800 ppm ergibt. Nach dem Durchmischen des Substrates lässt man das Aceton mindestens 20 Stunden lang verdampfen.

Dann werden pro Wirkstoff und Konzentration je 25 eintägige Maden von Musca domestica in die das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in mit Siebdeckeln verschlossenen Gefässen deponiert. Die pro Ansatz ausgeschwemmten Puppen werden gezählt (toxischer Einflusse des Wirkstoffes auf die Madenentwicklung). Dann wird nach 10 Tagen die Anzahl der aus den Puppen geschlüpften Fliegen bestimmt.

Verbindungen der Formeln I und II gemäss den Beispielen 1 bis 3 zeigen gute Wirkung im obigen Test.

Beispiel 8: Wirkung gegen Lucilia sericata:

Zu 9 ml eines Zuchtmediums wird bei 50 °C 1 ml einer 0,5 Gew.-% Aktivsubstanz enthaltenden wässrigen Zubereitung hinzugefügt. Nun werden ca. 30 frisch geschlüpfte Lucilia sericata-Larven zum Zuchtmedium gegeben, und nach 48 und 96 Stunden wird die insektizide Wirkung durch Ermittlung der Abtötungsrate festgestellt.

Verbindungen der Formeln I und II gemäss den Beispielen 1 bis 3 zeigen in diesem Test gute Wirkung gegen Lucilia sericata.

Beispiel 9: Wirkung gegen Lucilia cuprina

Frisch abgelegte Eier der Schmeissfliege L.cuprina werden in kleinen Portionen (30 - 50 Eier) in Reagenzgläser gegeben, in denen zuvor 4 ml Nährmedium mit 1 ml Wirkstofflösung vermischt worden sind. Nach der Beimpfung des Kulturmediums werden die Testgläser mit einem Wattestopfen verschlossen und im Brutschrank bei 30 °C über 4 Tage bebrütet.

In dem zum Vergleich unbehandelten Medium entwickeln sich bis zu diesem Zeitpunkt ca. 1 cm lange Larven (Stadium 3). Ist eine Substanz aktiv, so sind die Larven zu diesem Zeitpunkt entweder tot oder moribund. Der Versuch wird mit einer Wirkstoff-Konzentrationen von 400 ppm durchgeführt. Auch Repellenz wird berücksichtigt, wenn die Larven aus dem Medium auswandern und verhungern.

Verbindungen der Formeln I und II gemäss den Beispielen 1 bis 3 zeigen in diesem Test gute Wirkung gegen Lucilia cuprina.

Beispiel 10: Wirkung gegen Aëdes aegypti:

Auf die Oberfläche von 150 ml Wasser, das sich in einem Behälter befindet, wird so viel einer 0,1 Gew.-%igen acetonischen Lösung des Wirkstoffes pipettiert, dass eine Konzentration von 800 ppm erhalten wird. Nach Verdunsten des Acetons wird der Behälter mit 30 bis 40 2-tägigen Aëdes-Larven beschickt. Nach 1, 2 und 5 Tagen wird die Mortalität geprüft.

Verbindungen der Formeln I und II gemäss den Beispielen 1 bis 3 zeigen in diesem Test gute Wirkung gegen Aëdes aegypti.

Beispiel 11: Insektizide Frassgift-Wirkung:

Ca. 25 cm hohe eingetopfte Baumwollpflanzen werden mit wässrigen Wirkstoffemulsionen besprüht, die den Wirkstoff in Konzentrationen von 100, 200 und 400 ppm enthalten.

Nach dem Antrocknen des Sprühbelages werden die Baumwollpflanzen mit Spodoptera littoralis- bzw. Heliothis virescens-Larven im dritten larvalen Stadium besiedelt. Der Versuch wird bei 24 °C und 60 % relativer Luftfeuchtigkeit durchgeführt. Nach 2 Tagen wird die %-Mortalität der Larven bestimmt.

Eine Wirkung von 80-100 % (Mortalität) zeigen die erfindungsgemässen Verbindungen Nr. 1.1 und 1.2 bei 200 bzw. 400 ppm gegen Spodoptera-Larven. Verbindung Nr. 1.1 zeigt bei 100 ppm und die Verbindung Nr. 1.2 zeigt bei 400 ppm 80-100%ige Wirkung (Mortalität) gegen Heliothis-Larven.

Beispiel 12: Wirkung gegen Anthonomus grandis (Adulte)

Zwei eingetopfte Baumwollpflanzen im 6-Blattstadium werden jeweils mit einer wässrigen benetzungsfähigen Emulsions-Zubereitung enthaltend 400 ppm des zu prüfenden Wirkstoffes besprüht. Nach dem Antrocknen des Spritzbelages (nach etwa 1,5 Stunden) wird jede Pflanze mit 10 adulten Käfern (Anthonomus grandis) besiedelt. Plastikzylinder, deren obere Oeffnungen mit Gaze abgedeckt sind, werden dann über die behandelten, mit den Testtieren besiedelten Pflanzen gestülpt, um ein Abwandern der Käfer zu verhindern. Die so behandelten Pflanzen werden bei 25 °C und etwa 60 % relativer Luftfeuchtigkeit gehalten. Die Auswertung erfolgt nach 2, 3, 4 und 5 Tagen unter Zugrundelegung der prozentualen Mortalität der eingesetzten Testtiere (% Rückenlage) sowie der Antifeedant-Wirkung gegenüber unbehandelten Kontrollansätzen.

Verbindungen der Formel I und II gemäss Beispielen I bis 3 zeigen gute Wirkung in obigem Test.

Beispiel 13: Wirkung gegen Zecken

A) Amblyomma hebraeum

50 Nymphen werden in ein Glasröhrchen gezählt und für 1 bis 2 Minuten in 2 ml einer wässrigen

Emulsion enthaltend 400 ppm Testsubstanz getaucht. Das Röhrchen wird dann mit einem genormten Wattebausch verschlossen und auf den Kopf gestellt, damit die Wirkstoffemulsion von der Watte aufgenommen werden kann.

Die Auswertung erfolgt nach 1 Woche. Für jeden Versuch werden 2 Wiederholungen durchgeführt.

B) Boophilus microplus (Larven)

Mit einer analogen Verdünnungsreihe wie beim Test A) werden mit je 20 sensiblen resp. OP-resistenten Larven Versuche durchgeführt. (Die Resistenz bezieht sich auf die Verträglichkeit von Diazinon).

Die Verbindungen der Formeln I und II gemäss den Beispielen 1 bis 3 zeigen in diesem Test gute Wirkung gegen Nyumphen bzw. Larven der Zecken Amblyomma hebraeum und Boophilus microplus.

Beispiel 14: Insektizide Wirkung: Nilaparvata lugens

Reispflanzen werden mit einer Versuchslösung, enthaltend 400 ppm der zu prüfenden Verbindung, besprüht. Nach dem Antrocknen des Belages werden die Pflanzen mit Nymphen von Nilaparvata lugens (N2 oder N3-Stadium) besetzt. Man verwendet pro Versuchsverbindung und pro Test-Spezies zwei Pflanzen. Die Auswertung der erzielten prozentualen Abtötungsrate erfolgt nach 6 Tagen. Der Versuch wird bei 26 ° C und 60 % relativer Luftfeuchtigkeit durchgeführt.

Verbindung Nr. 1.1 zeigt im obigen Test 80-100%-ige Wirkung gegenüber Niaparvata lugens-Nymphen.

Beispiel 15: Wirkung gegen Bodenisnekten (Diabreotica balteata)

Es werden 5 Maiskeimlinge von 1-3 cm sowie eine Filterpapier-Rondelle in eine wässrige Wirkstofflösung enthaltend etwa 4 Vol.-% Aceton eingetaucht. Die eingetauchte Filterpapier-Rondelle wird auf den Boden eines Konststoff-Bechers (Inhalt 200 ml) gelegt und darauf word eine trockene Filterpapier-Rondelle sowie die Maiskeimlinge und 10 Larben von Diabrotica balteata im 2. oder 3. Larvalstadium gegeben. Der Ansatz wird bei ca. 24 ° C und 40-60 % relativer Luftfeuchtigkeit und Tagesli8cht gehalten. Die Bonitur erfolgt nach 6 Tagen gegenüber unbehandelten Kontrollansätzen.

Die erfindungsgemässe Verbindung Nr. 1.15 zeigt in diesem Test bei 400 ppm eine Wirkung von 80-100 % (Mortalität).

Beispiel 16: Wirkung gegen Nephotettix cincticeps (Nymphen)

Der Test wird an wachsenden Pflanzen durchgeführt. Dazu werden ca. 20 Tage alte Reispflanzen mit einer Höhe von etwa 15 cm in Töpfe (Durchmesser 5,5 cm) eingepflanzt.

Die Pflanzen werden auf einem Drehteller mit jeweils 100 ml einer acetonischen Lösung enthaltend 400 ppm des zu prüfenden Wirkstoffs besprüht. Nach dem Antrocknen des Spritzbelages erfolgt die Besiedlung jeder Pflanze mit je 20 Nymphen der Testtiere im zweiten oder dritten Stadium. Um die Zikaden am Entweichen zu hindern, wird über die besiedelten Pflanzen jeweils ein Plexiglaszylinder gestülpt und dieser mit einem Gaze-Deckel abgedeckt. Die Nymphen werden für 5 Tage an der behandelten Pflanze, die mindestens 1 mal nachgegossen werden muss, gehalten. Der Versuch wird bei einer Temperatur von ca. 23 ° C, bei 55 % relativer Luftfeuchtigkeit und mit einer Belichtungsperiode von 16 Stunden durchgeführt.

Die erfindungsgemässe Verbindung Nr. 1.1 gemäss Beispiel 1 zeigt in diesem Test 80-100 %-ige Wirkung.

Beispiel 17: Insektizide Frassgift-Wirkung gegen Plutella xylostella:

Eingetopfte Chinakohlpflanzen (Topfgrösse 10 cm Durchmesser) im 4-Blatt-Stadium werden mit wässrigen Wirkstoffemulsionen besprüht, die den Wirkstoff in Konzentrationen von 3 bis 400 ppm enthalten und auf den Pflanzen antrocknen.

Nach zwei Tagen werden die behandelten Chinakohlpflanzen mit je 10 Platella xylostella-Larven im zweiten larvalen Stadium besiedelt. Der Versuch wird bei 24 ° C und 60 % relativer Luftfeuchtigkeit im Dämmerlicht durchgeführt. Die Bontitur erfolgt nach 2 und 5 Tagen; es wird die %-Mortalität der Larven bestimmt.

Eine Wirkung von 80-100 % (Mortalität) zeigen die Verbindungen der Formel I gemäss Beispiel 1 bis 4 in diesem Test.

## Patentansprüche

**1.** Stabile Verbindung der Formel

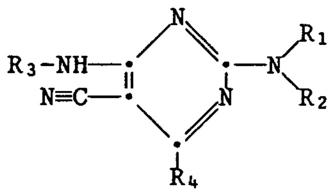

(I),

worin

| | |
|---|---|
| $R_1$ | Wasserstoff, $C_1$-$C_6$-Alkyl, $C_2$-$C_6$-Alkenyl oder $C_2$-$C_6$-Alkinyl; |
| $R_2$ | Wasserstoff, $C_1$-$C_{10}$-Alkyl oder $C_3$-$C_6$-Cycloalkyl; oder |
| $R_1$ und $R_2$ | zusammen einen der Reste $-(CH_2)_3-$, $-(CH_2)_4-$ oder $-(CH_2)_5-$; $R_3$ Wasserstoff oder einen der Reste $-CO-R_5$ oder $-SO_2-R_6$ ; |
| $R_4$ | einen der Reste $-NH_2$, $-NH-CO-R_5$, $-NH-SO_2-R_6$ , 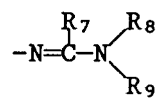 oder $-NH-CH=N-R_{10}$ ; |
| $R_5$ | Wasserstoff, $C_1$-$C_{12}$-Alkyl, $C_3$-$C_6$-Cycloalkyl, $C_1$-$C_6$-Alkoxy, perhalogenierte $C_1$-$C_3$-Alkyl oder den Rest 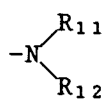 ; |
| $R_6$ | $C_1$-$C_6$-Alkyl; |
| $R_7$ | Wasserstoff oder $C_1$-$C_6$-Alkyl; |
| $R_8$ und $R_9$ | unabhängig voneinander Wasserstoff oder $C_1$-$C_6$-Alkyl oder zusammen einen der Reste $-(CH_2)_3-$, $-(CH_2)_4-$ oder $-(CH_2)_5-$; |
| $R_{10}$ | einer der Reste $-SO_2-R_{13}$ oder 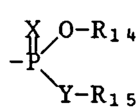 ; |
| $R_{11}$ und $R_{12}$ | unabhängig voneinander Wasserstoff oder $C_1$-$C_6$-Alkyl oder zusammen einen der Reste $-(CH_2)_3-$, $-(CH_2)_4-$ oder $-(CH_2)_5-$; |
| $R_{13}$ | $C_1$-$C_{10}$-Alkyl, mit bis zu 10 Halogenatomen substituiertes $C_1$-$C_{10}$-Alkyl oder $C_3$-$C_6$-Cycloalkyl; |
| $R_{14}$ und $R_{15}$ | unabhängig voneinander $C_1$-$C_{10}$-Alkyl; |
| X und Y | unabhängig voneinander Sauerstoff oder Schwefel bedeuten, sowie deren Salze. |

**2.** Verbindung gemäss Anspruch 1 der Formel I, dadurch gekennzeichnet, dass

| | |
|---|---|
| $R_1$ | Wasserstoff oder $C_1$-$C_4$-Alkyl; |
| $R_2$ | $C_1$-$C_4$-Alkyl oder $C_3$-$C_6$-Cycloalkyl oder |

$R_2$ und $R_2$ zusammen einen der Reste $-(CH_2)_4-$ oder $-(CH_2)_5-$;
$R_3$ Wasserstoff;
$R_4$ einen der Reste $-NH_2$ oder $-NH-CO-R_5$; und
$R_5$ $C_1$-$C_4$-Alkyl oder perhalogeniertes $C_1$-$C_3$-Alkyl bedeuten, sowie deren Salze.

**3.** Verbindung gemäss Anspruch 2 der Formel I, dadurch gekennzeichnet, dass
$R_1$ Wasserstoff, Methyl oder Aethyl;
$R_2$ $C_1$-$C_4$-Alkyl oder Cyclopropyl;
$R_3$ Wasserstoff;
$R_4$ einen der Reste $-NH_2$ oder $-NH-CO-R_5$;
$R_5$ $C_1$-$C_3$-Alkyl bedeuten, sowie deren Salze.

**4.** Verbindung gemäss Anspruch der Formel I, dadurch gekennziechnet, dass $R_1$ Wasserstoff, Methyl oder Aethyl;
$R_2$ Cyclopropyl;
$R_3$ Wasserstoff; und
$R_4$ einen der Reste $-NH_2$ oder $-NH-CO-C_2H_5$ bedeuten, sowie deren Salze.

**5.** Verbindung gemäss Anspruch 4 der Formel

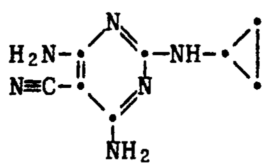

**6.** Verbindung gemäss Anspruch 4 der Formel

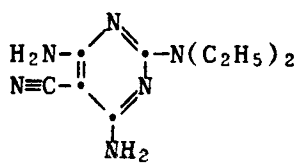

**7.** Verbindung gemäss Anspruch 4 der Formel

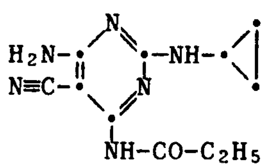

**8.** Verbindung gemäss Anspruch 4 der Formel

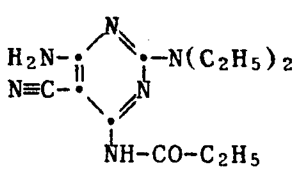

**9.** Verfahren zur Herstellung einer stabilen Verbindung der Formel I gemäss einem der Ansprüche 1 bis 8 sowie deren Salze, dadurch gekennzeichnet, dass man

a) eine Verbindung der Formel II

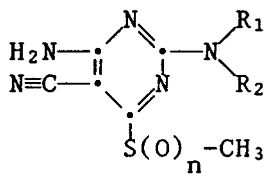

(II),

mit Ammoniak oder

b) die Verbindung der Formel III

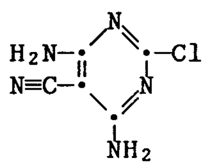

(III)

mit einer Verbindung der Formel IV

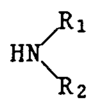

(IV)

umsetzt und die jeweils entstandene Verbindung der Formel I, worin $R_3$ Wasserstoff und $R_4$ den Rest $-NH_2$ bedeuten, gegebenenfalls in an sich bekannter Weise in eine Verbindung der Formel I überführt, worin $R_3$ die Bedeutungen $-CO-R_5$ oder $-SO_2-R_6$ hat und/oder $R_4$ die Bedeutungen $-NH-CO-R_5$, $-NH-SO_2-R_6$,

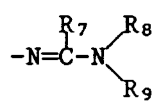

oder $-NH-CH=N-R_{10}$ hat,

wobei $R_1$ bis $R_{15}$, X und Y die in Anspruch 1 angegebenen Bedeutungen haben sowie n eine Zahl 0, 1 oder 2 bedeutet, und dass man erwünschtenfalls eine erhaltene Verbindung der Formel I in eines ihrer Salze überführt.

**10.** Stabile Verbindung der Formel II

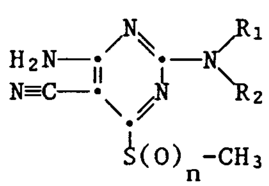

(II),

worin

$R_1$ Wasserstoff, $C_1$-$C_6$-Alkyl, $C_2$-$C_6$-Alkenyl oder $C_2$-$C_6$-Alkinyl;

| | |
|---|---|
| $R_2$ | Wasserstoff, $C_1$-$C_{10}$-Alkyl oder $C_3$-$C_6$-Cycloalkyl; oder |
| $R_1$ und $R_2$ | zusammen einen der Reste $-(CH_2)_4-$ oder $-(CH_2)_5-$; |
| n | eine Zahl 0, 1 oder 2 bedeuten. |

**11.** Verfahren zur Herstellung der Verbindung der Formel III, dadurch gekennzeichnet, dass man die Verbindung der Formel VII

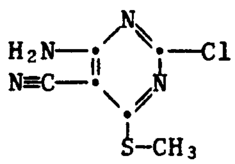

(VII),

mit Chlor zu der Verbindung der Formel XIII

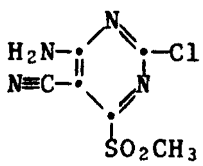

(XIII)

oxydiert und die Verbindung der Formel XIII mit Ammoniak umsetzt.

**12.** Schädlingsbekämpfungsmittel, welches als aktive Komponente eine Verbindung der Formeln I oder II gemäss einem der Ansprüche 1 bis 8 oder 10 oder eines ihrer Salze zusammen mit geeigneten Trägern und/oder anderen Zuschlagstoffen enthält.

**13.** Verwendung einer Verbindung gemäss einem der Ansprüche 1 bis 8 oder 10 oder eines ihrer Salze zur Bekämpfung von Insekten und Vertretern der Ordnung Akarina an Tieren und Pflanzen.

**14.** Verwendung gemäss Anspruch 13 zur Bekämpfung larvaler Stadien pflanzenschädigender Insekten.

**15.** Verwendung gemäss Anspruch 13 zur Bekämpfung von Ektoparasiten an Haus- und Nutztieren.

**16.** Verfahren zur Bekämpfung von Insekten und Vertretern der Ordnung Akarina, dadurch gekennzeichnet, dass man diese Schädlinge bzw. deren verschiedene Entwicklungsstadien oder deren Aufenthaltsort mit einer pestizid wirksamen Menge einer Verbindung der Formel I gemäss einem der Ansprüche 1 bis 8 oder 10 oder eines ihrer Salze oder mit einem Mittel enthaltend neben Zusatz-und Trägerstoffen eine pestizid wirksame Menge dieser Verbindung, in Kontakt bringt oder behandelt.

**Claims**

**1.** A stable compound of the formula

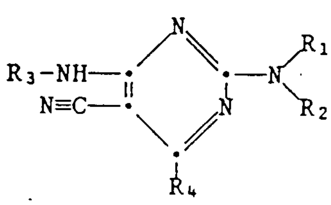

(I),

wherein

| | |
|---|---|
| $R_1$ | is hydrogen, $C_1$-$C_6$alkyl, $C_2$-$C_6$alkenyl or $C_2$-$C_6$alkynyl, |
| $R_2$ | is hydrogen, $C_1$-$C_{10}$alkyl or $C_3$-$C_6$cycloalkyl, or |
| $R_1$ and $R_2$, | when taken together, are one of the radicals $-(CH_2)_3-$, $-(CH_2)_4-$or $-(CH_2)_5-$, |

$R_3$ is hydrogen or one of the radicals $-CO-R_5$ or $-SO_2-R_6$,
$R_4$ is one of the radicals
$-NH_2$, $-NH-CO-R_5$, $-NH-SO_2-R_6$,

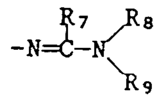

or $-NH-CH=N-R_{10}$ ;
$R_5$ is hydrogen, $C_1$-$C_{12}$alkyl, $C_3$-$C_6$cycloalkyl, $C_1$-$C_6$alkoxy, perhalogenated $C_1$-$C_3$alkyl or the radical

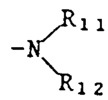 ;

$R_6$ is $C_1$-$C_6$alkyl,
$R_7$ is hydrogen or $C_1$-$C_6$alkyl,
$R_8$ and $R_9$ are each independently of the other hydrogen or $C_1$-$C_6$alkyl or, when taken together, are one of the radicals $-(CH_2)_3-$, $-(CH_2)_4-$ or $-(CH_2)_5-$,
$R_{10}$ is one of the radicals $-SO_2-R_{13}$ or

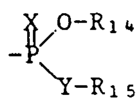 ;

$R_{11}$ and $R_{12}$ are each independently of the other hydrogen or $C_1$-$C_6$alkyl or, when taken together, are one of the radicals $-(CH_2)_3-$, $-(CH_2)_4-$ or $-(CH_2)_5-$,
$R_{13}$ is $C_1$-$C_{10}$alkyl, $C_1$-$C_{10}$alkyl which is substituted by up to 10 halogen atoms, or is $C_3$-$C_6$cyclolkyl,
$R_{14}$ and $R_{15}$ are each independently of the other $C_1$-$C_{10}$alkyl; and
X and Y are each independently of the other oxygen or sulfur, and salts thereof.

**2.** A compound according to claim 1 of the formula I,
wherein
$R_1$ is hydrogen or $C_1$-$C_4$alkyl,
$R_2$ is $C_1$-$C_4$alkyl or $C_3$-$C_6$cycloalkyl, or
$R_1$ and $R_2$, when taken together, are one of the radicals $-(CH_2)_4-$ or $-(CH_2)_5-$,
$R_3$ is hydrogen,
$R_4$ is one of the radicals $-NH_2$ or $-NH-CO-R_5$, and
$R_5$ is $C_1$-$C_4$alkyl or perhalogenated $C_1$-$C_3$alkyl, and salts thereof.

**3.** A compound according to claim 2 of the formula I,
wherein
$R_1$ is hydrogen, methyl or ethyl,
$R_2$ is $C_1$-$C_4$alkyl or cyclopropyl,
$R_3$ is hydrogen,
$R_4$ is one of the radicals $-NH_2$ or $-NH-CO-R_5$, and
$R_5$ is $C_1$-$C_3$alkyl, and salts thereof.

**4.** A compound according to claim 3 of the formula I,
wherein
$R_1$ is hydrogen, methyl or ethyl,

$R_2$ is cyclopropyl,
$R_3$ is hydrogen, and
$R_4$ is one of the radicals $-NH_2$ or $-NH-CO-C_2-H_5$, and salts thereof.

**5.** A compound according to claim 4 of the formula

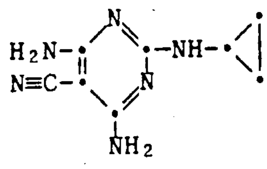

**6.** A compound according to claim 4 of the formula

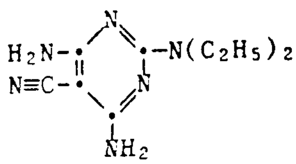

**7.** A compound according to claim 4 of the formula

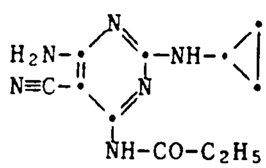

**8.** A compound according to claim 4 of the formula

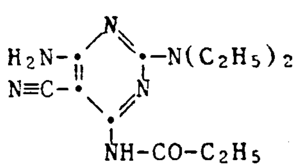

**9.** A process for the preparation of a stable compound according to any one of claims 1 to 8, and salts thereof, which comprises

a) reacting a compound of the formula II

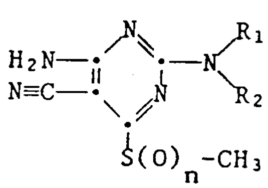

(II),

with ammonia, or

b) the compound of the formula III

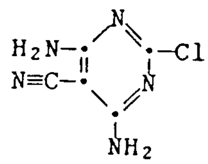

(III)

with a compound of the formula IV

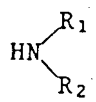

(IV)

and, if desired, converting the particular resultant compound of the formula I, in which $R_3$ is hydrogen and $R_4$ is the radical $-NH_2$, in a manner known per se, into a compound of the formula I, in which $R_3$ has the meanings $-CO-R_5$ or $-SO_2-R_6$ and/or $R_4$ has the meanings $-NH-CO-R_5$, $-NH-SO_2-R_6$,

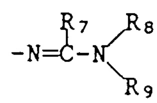

or $-NH-CH=N-R_{10}$,

where $R_1$ to $R_{15}$, X and Y are as defined in claim 1 and n is 0, 1 or 2, and, if desired, converting a resultant compound of the formula I into a salt thereof.

**10.** A stable compound of the formula II

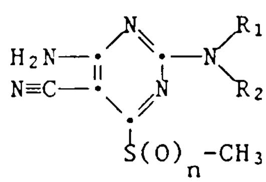

(II),

wherein

| | |
|---|---|
| $R_1$ | is hydrogen, $C_1$-$C_6$alkyl, $C_2$-$C_6$alkenyl or $C_2$-$C_6$alkynyl, |
| $R_2$ | is hydrogen, $C_1$-$C_{10}$alkyl or $C_3$-$C_6$cycloalkyl, or |
| $R_1$ and $R_2$, | when taken together, are one of the radicals $-(CH_2)_4-$ or $-(CH_2)_5-$, and |
| n | is 0, 1 or 2. |

**11.** A process for the preparation of a compound of the formula III, which comprises oxidising the compound of the formula VII

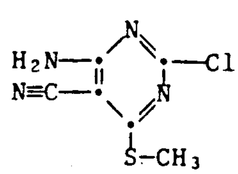

(VII)

with chlorine, to give the compound of the formula XIII

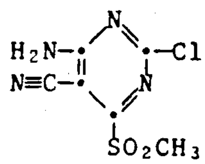

(XIII)

and reacting the compound of the formula XIII with ammonia.

**12.** A pesticide which contains, as active component, a compound of the formula I or II according to any one of claims 1 to 8 or 10, or a salt thereof, together with suitable carriers and/or other adjuvants.

**13.** Use of a compound according to any one of claims 1 to 8 or 10, or a salt thereof, for controlling insects and representatives of the order Acarina on animals or plants.

**14.** Use according to claim 13 for controlling larval stages of plant-destructive insects.

**15.** Use according to claim 13 for controlling ectoparasites in domestic animals and productive livestock.

**16.** Method of controlling insects and representatives of the order Acarina, which comprises treating or contacting these pests, their various development stages or the locus thereof with a pesticidally effective amount of a compound of the formula I according to any one of claims 1 to 8 or 10, or of a salt thereof, or with an agent comprising a pesticidally effective amount of this compound, together with adjuvants and carriers.

**Revendications**

**1.** Composé stable de formule

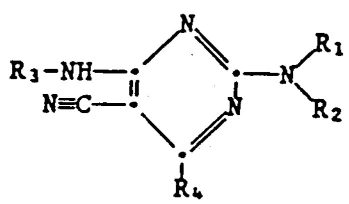

(I),

où

$R_1$ représente un hydrogène, un alkyle en $C_1$-$C_6$, un alcényle en $C_2$-$C_6$ ou un alcynyle en $C_2$-$C_6$;

$R_2$ représente un hydrogène, un alkyle en $C_1$-$C_{10}$ ou un cycloalkyle en $C_3$-$C_6$; ou

$R_1$ et $R_2$ représentent ensemble un des restes $-(CH_2)_3-$, $-(CH_2)_4-$ ou $-(CH_2)_5$;

$R_3$ représente un hydrogène ou un des restes $-CO-R_5$ ou $-SO_2-R_6$,

$R_4$ représente un des restes $-NH_2$, $-NH-CO-R_5$, $-HH-SO_2-R_6$ ,

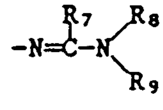

ou $-NH-CH=N-R_{10}$ ;

$R_5$ représente un hydrogène, un alkyle en $C_1$-$C_{12}$, un cycloalkyle en $C_3$-$C_6$, un alcoxy en $C_1$-$C_6$, un alkyle en $C_1$-$C_3$ perhalogéné ou le reste

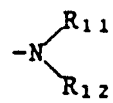 ;

$R_6$ représente un alkyle en $C_1$-$C_6$;
$R_7$ représente un hydrogène ou un alkyle en $C_1$-$C_6$;
$R_8$ et $R_9$, indépendamment l'un de l'autre, représentent un hydrogène ou un alkyle en $C_1$-$C_6$ ou ensemble un des restes $-(CH_2)_3-$, $-(CH_2)_4-$ ou $-(CH_2)_5-$;
$R_{10}$ représente un des restes $-SO_2-R_{13}$ ou

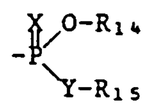 ;

$R_{11}$ et $R_{12}$ indépendamment l'un de l'autre représentent un hydrogène ou un alkyle en $C_1$-$C_6$ ou ensemble un des restes $-(CH_2)_3-$, $-(CH_2)_4-$ ou $-(CH_2)_5-$;
$R_{13}$ représente un alkyle en $C_1$-$C_{10}$, un alkyle en $C_1$-$C_{10}$ substitué avec jusqu'à 10 atomes d'halogène ou un cycloalkyle en $C_3$-$C_6$;
$R_{14}$ et $R_{15}$ indépendamment l'un de l'autre représentent un alkyle en $C_1$-$C_{10}$;
X et Y indépendamment l'un de l'autre représentent un oxygène ou un soufre,
ainsi que ses sels.

**2.** Composé selon la revendication 1 de formule I, caractérisé en ce que
$R_1$ représente un hydrogène ou un alkyle en $C_1$-$C_4$;
$R_2$ représente un alkyle en $C_1$-$C_4$ ou un cycloalkyle en $C_3$-$C_6$ ou
$R_1$ et $R_2$ ensemble représentent un des restes $-(CH_2)_4-$ou $-(CH_2)_5-$;
$R_3$ représente un hydrogène;
$R_4$ représente un des restes $-NH_2$ ou $-NH-CO-R_5$; et
$R_5$ représente un alkyle en $C_4$-$C_4$ ou un alkyle en $C_1$-$C_3$ perhalogéné,
ainsi que ses sels.

**3.** Composé selon la revendication 2 de formule I, caractérisé en ce que
$R_1$ représente un hydrogène, un méthyle ou un éthyle;
$R_2$ représente un alkyle en $C_1$-$C_4$ ou un cyclopropyle;
$R_3$ représente un hydrogène;
$R_4$ représente un des restes $-NH_2$ ou $-NH-CO-R_5$;
$R_5$ représente un alkyle en $C_1$-$C_3$,
ainsi que ses sels.

**4.** Composé selon la revendication 3 de formule I, caractérisé en ce que
$R_1$ représente un hydrogène, un méthyle ou un éthyle;
$R_2$ représente un cyclopropyle;
$R_3$ représente un hydrogène; et
$R_4$ représente un des restes $-NH_2$ ou $-NH-CO-C_2H_5$,
ainsi que ses sels.

**5.** Composé selon la revendication 4 de formule

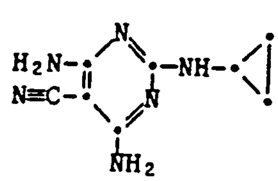

6. Composé selon la revendication 4 de formule

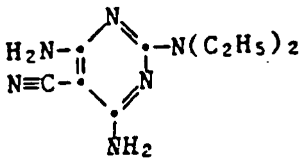

7. Composé selon la revendication 4 de formule

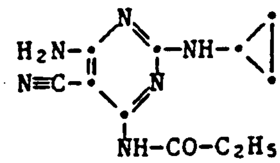

8. Composé selon la revendication 4 de formule

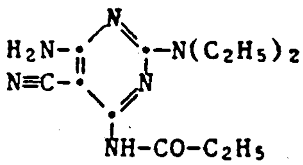

9. Procédé pour préparer un composé stable de formule I selon l'une des revendications 1 à 8, ainsi que ses sels, caractérisé en ce que l'on fait réagir
a) un composé de formule II

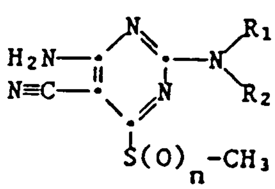

(II),

avec l'ammoniac, ou on fait réagir
b) le composé de formule III

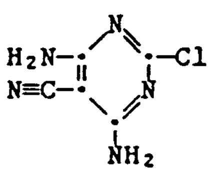

(III)

avec un composé de formule IV

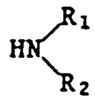

(IV)

et en ce que l'on transforme le composé de formule I qui se forme à chaque fois, dans lequel $R_3$ représente un hydrogène et $R_4$ représente le reste $-NH_2$, éventuellement, de façon connue en soi, en un composé de formule I où $R_3$ a les significations $-CO-R_5$ ou $-SO_2-R_6$ et/ou $R_4$ a les significations $-NH-CO-R_5$, $-NH-SO_2-R_6$,

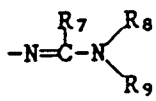

ou $-NH-CH=N-R_{10}$,
où $R_1$ à $R_{15}$, X et Y ont les significations données à la revendication 1, et n est un nombre égal à 0, 1 ou 2, et en ce que, si on le désire, on transforme un composé obtenu de formule I en un de ses sels.

**10.** Composé stable de formule II

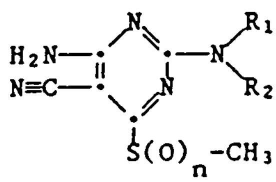

(II),

où

| | |
|---|---|
| $R_1$ | représente un hydrogène, un alkyle en $C_1$-$C_6$, un alcényle en $C_2$-$C_6$ ou un alcynyle en $C_2$-$C_6$; |
| $R_2$ | représente un hydrogène, un alkyle en $C_1$-$C_{10}$ ou un cycloalkyle en $C_3$-$C_6$; ou |
| $R_1$ et $R_2$ | représentent ensemble un des restes $-(CH_2)_4$-ou $-(CH_2)_5$-; |
| n | représente un nombre égal à 0, 1 ou 2. |

**11.** Procédé pour préparer le composé de formule III, caractérisé en ce que l'on oxyde le composé de formule VII

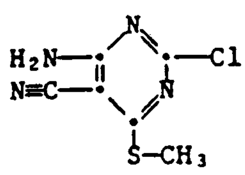

(VII),

avec le chlore pour obtenir le composé de formule XIII

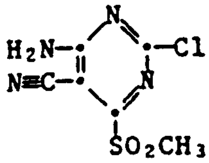

(XIII)

et en ce que l'on fait réagir le composé de formule XIII avec de l'ammoniac.

**12.** Moyen pour lutter contre les parasites, qui contient comme composant actif un composé des formules I ou II conforme à l'une des revendications 1 à 8 ou 10, ou un de ses sels, en même temps que des supports appropriés et/ou d'autres additifs appropriés.

**13.** Utilisation d'un composé selon l'une des revendications 1 à 8 ou 10, ou un de ses sels, pour lutter contre les insectes et les représentants de l'ordre de l'acare sur les animaux et les plantes.

**14.** Utilisation selon la revendication 13 pour lutter contre les insectes au stade de larves qui sont nuisibles pour les plantes.

**15.** Utilisation selon la revendication 13 pour luttter contre les ectoparasites sur les animaux de compagnie et les animaux utiles.

**16.** Procédé pour lutter contre les insectes et les représentants de l'ordre de l'acare, caractérisé en ce que l'on traite ces parasites, ou leurs divers stades d' évolution ou leurs lieux de séjour, avec une quantité efficace comme pesticide d'un composé de formule I selon l'une des revendications 1 à 8 ou 10, ou d'un de ses sels, ou avec un moyen contenant, en plus des additifs et des supports, une quantité efficace comme pesticide, de ce composé, ou en ce qu'on les met au contact de ladite quantité ou dudit moyen.